# EUROPÄISCHE PATENTANMELDUNG

(11) **EP 1 148 770 A2**
(43) Veröffentlichungstag der Anmeldung: **24.10.2001**
(21) Anmeldenummer: 01104487.2
(22) Anmeldetag: 01.03.2001
(51) Int. Cl.: H05H 1/36

(54) **Plasmagenerator für die HF-Chirurgie**

(30) Priorität: 21.04.2000 US 557906
(71) Anmelder: Söring GmbH, 25451 Quickborn (DE)
(72) Erfinder: Soll, Joachim, 25421 Pinneberg (DE); Zobawa, Klaus, 25337 Elmshorn (DE); Trinh, Buu Son, 22307 Hamburg (DE); Söring, Holger, 25451 Quickborn (DE)

(57) **Zusammenfassung**

Die vorliegende Erfindung ist ein Plasmagenerator zur Erzeugung eines kalten Plasmastrahls zur Anwendung in der Medizin, besonders in der Chirurgie sowie in anderen Bereichen der Wissenschaft. Der Plasmagenerator wird von einem separaten, frequenz-variablen Oszillator gespeist, der die Leistungsendstufe über eine Treiberstufe steuert. Die vom Generator erzeugte Hochfrequenz wird durch eine Resonanzfrequenz eines Resonanzübertragers bestimmt. Der Plasmastrom wird durch einen in einem Handstück befindlichen Kondensator begrenzt.

## Beschreibung

Die vorliegende Erfindung bezieht sich auf einen Plasmagenerator zur Erzeugung eines kalten Plasmastrahls für die Anwendung im medizinischen Bereich, insbesondere für chirurgische Anwendungen, sowie für andere Wissenschaftsbereiche.

Plasmaquellen, die unter atmosphärischem Druck ein Plasma von mehr als 1000 Grad erzeugen können, sind allgemein üblich. Die Erwärmung des Plasmas geschieht durch konventionelle elektromagnetische Induktion, genauer: durch die induktive Erwärmung von elektrisch leitenden Medien im elektromagnetischen Wechselfeld einer Induktionsspule.

Diese Plasmaquelle besteht aus einem Wechselstrom-Hochspannungsgenerator, einem röhrenförmigen Quarzbehälter und einer flüssigkeitsgekühlten Induktionsspule mit einer hohen Anzahl Windungen. Eine Spule mit einem Durchmesser von 10 cm ist unbedingt erforderlich, um eine Induktion in einem Volumen von 1 Liter zu erzeugen. Der Induktionswiderstand steigt bei Frequenzen von über 1 MHz sehr stark an. Im allgemeinen ist es schwierig, den Hochspannungsgenerator an den Induktionswiderstand anzupassen, da die effektive Ausgangsleistung dadurch verringert wird. Die Entwicklung einer Plasmaquelle nach den Erkenntnissen der Technik führt zu einer Verkleinerung der äußeren Abmessungen, aber auch zu einem geringeren Wirkungsgrad und zu höheren Plasmatemperaturen, was, insbesondere in den Bereichen der Medizin, Biologie und der Umweltanwendungen, den Einsatzbereich einschränkt.

Andere Plasmageneratoren werden in elektrischen Entladungsapparaten verwendet, die mit einem elektronischen Oszillator, einer Modulationsstufe, einem Resonanzübertrager sowie einer stabförmigen Entladungselektrode ausgestattet sind. Ein Plasmastrahl wird hier zwischen dem Elektrodenende und dem Objekt erzeugt. Der elektronische Oszillator erzeugt Schwingungen in der Größenordnung von ca. 200 bis 300 kHz. Der Modulator schaltet das Oszillatorsignal in einem Frequenzbereich von 3 bis 5 kHz an und aus. Auf diese Weise sorgt der Modulator für eine verstärkte Erregung des Resonanzübertrager und des Objekts. Dieser Apparat hat einen günstigen therapeutischen Effekt bei der Behandlung von vielen Krankheiten, indem er verschiedenen Teilen des Körpers Wärme zuführt.

Dieser Apparat besitzt jedoch nachteilige Eigenschaften: so wird die Umwandlung der Entladung von der Korona zur Funkengestalt sichtbar, und dabei führt der Leiter direkt in das Gewebe.

Die klassische Korona- und Funkenentladung führt unweigerlich zur Ionisierung, elektrischen Zerstörung und Erwärmung des Luftspalts zwischen der Elektrode und dem Körper, der die zweite Elektrode darstellt. Dadurch kommt es zu einer durch die Wärme verursachten, chaotischen Bewegung der Partikel in der Entladungszone.

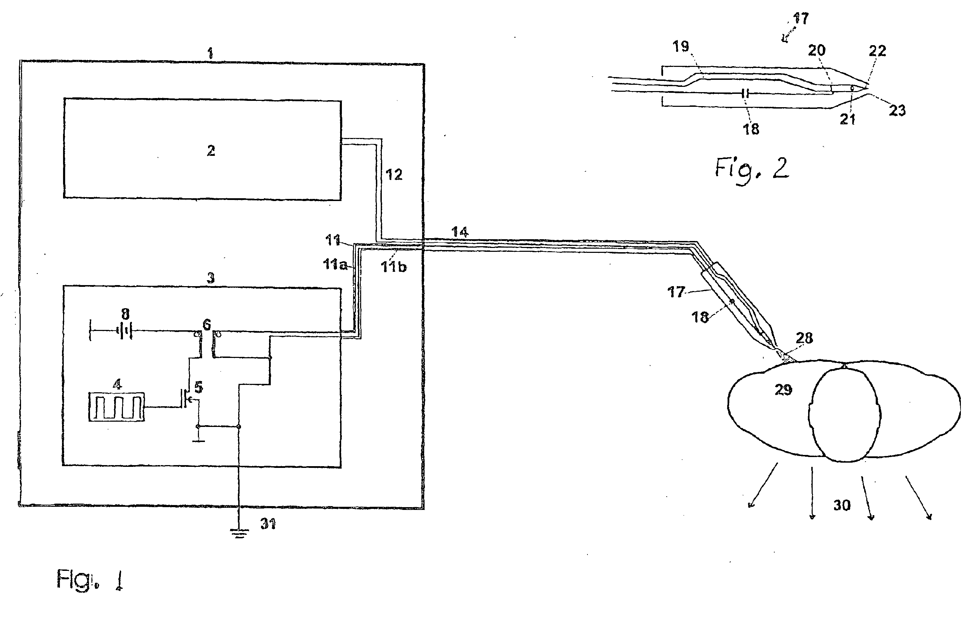

Die herkömmliche HF-Chirurgie verwendet einen HF-Frequenzgenerator für die Spraykoagulation oder Argonkoagulation (Plasmaentladung innerhalb eines Argonstrahls). In diesem Fall arbeitet der HF-Generator gemäß dem Prinzip der Stoßerregung von Schwingendkreisen. Diese Impulserregung eines Schwingkreises erzeugt kurze HF-Impulse (Bursts) mit einem, gemessen an der Impulsdauer langen, Pausenintervall (siehe Abb. 2). Das Verhältnis Impulsdauer/Pausenintervall beträgt in der Regel zwischen 10 und 20 Prozent. Dadurch kommt es zu starken Spitzenleistungen während der Bursts und folglich zu sehr hohen Spitzenströmen, die mehrere Ampere erreichen können. Die Kapazität zwischen Patient und Erde ist unter diesen Umständen nicht ausreichend, um den Stromkreis zu schließen und macht daher in der Regel bei konventioneller HF-Chirurgie die Verwendung einer neutralen Elektrode nötig. Außerdem wird die Kontaktfläche des Entladungsbogens auf das Gewebe relativ groß und kann mehrere Quadratmillimeter erreichen, so daß die Durchdringungstiefe des Hitzeeffekts als sehr groß empfunden wird.
Außerdem führen Stromspitzen, die durch das Gewebe strömen, auch in tieferen Bereichen zu einer hohen Strombelastung, die auf empfindliches Gewebe, wie z.B. Nervengewebe im Gehirn, schädigend wirken kann. Das Spektrum eines HF-Stroms, der aus kurzen Bursts besteht, enthält immer auch einen nicht zu unterschätzenden Faktor, der sich aus der Pulswiederholfrequenz herleitet. Diese Frequenz liegt üblicherweise zwischen 10 und 70 kHz. Diese tiefen Frequenzen können Schäden an empfindlichem Gewebe verursachen. Plasmageneratoren herkömmlicher Bauart, die das Plasma auf induktivem Weg oder über eine Bogenentladung erzeugen, sind aufgrund ihrer Kühlanforderungen für ein Handstück zu schwer. Außerdem erzeugen konventionelle Plasmageneratoren sehr hohe Plasmatemperaturen von mehreren Tausend Grad Celsius als Wärmeplasma.

Ein weiterer Plasmagenerator wird in dem europäischen Patentdokument EP-A 0837622 beschrieben. Dieser Generator enthält eine Spannungsquelle und einen elektrischen Oszillator, wobei der elektrische Oszillator von einem Verstärker versorgt wird, der an ein Niedervoltgerät angeschlossen ist, einem Resonanzübertrager mit einem Niederspannungseingang und einem Hochspannungsausgang und einer Verbindung des Hochspannungsausgangs des Resonanzübertragers mit der Entladungselektrode. Dieser Oszillator besitzt eine Rückkopplungswicklung, an der ein einstufiger Leistungsoszillator angeschlossen ist. Die sehr geringe Stabilität der Entladungsamplitude und die damit verbundenen Probleme der Leistungssteuerung sind in Zusammenhang mit einem solchen einstufigen Leistungsoszillator von Nachteil.

Das US-Patent 4,781,175 (ausgestellt auf McGreevy und Andere) beschreibt die Anwendung eines elektrochirurgischen leitenden Gasstrahls zur Verbesserung der Schorfbildung für die Gerinnung. Ein vorab bestimmtes ionisierbares Gas wird dem Gewebe strahlförmig bei einer Durchflußmenge zugeführt, die zur Beseitigung natürlicher Flüssigkeiten vom Gewebe und zur Offenlegung des Gewebestroma ausreichend ist. Um eine Austrocknung zu erreichen, wird elektrische Energie als Lichtbögen in den ionisierten leitfähigen Bereichen zugeführt.

Die europäische Patentanmeldung EP 0787465 A1 (ausgestellt auf Kim und Andere) beschreibt einen Kaltplasmakoagulator. Dieser Kaltplasmakoagulator besteht aus einem Hochfrequenznetzteil, einem Gasdynamikblock und einem Plasmotron. Das Netzteil besteht aus Gleichrichter, Kondensatorpuffer und einem Spannungsinverter. Ein Resonanzinduktor sowie ein nicht leitendes Rohr sind koaxial angeordnet, wobei das eine Ende des nicht leitenden Rohrs mit dem Gasdynamikblocks verbunden ist, während das andere Ende durch eine Düse Plasma ausstößt. Die Windungen des Resonanzinduktors bestehen aus einem Niederspannungs- und einem Hochspannungsteil. Das Niederspannungsteil der Resonanzspulen ist mit dem Ausgang des Spannungsinverters verbunden.

Das DDR-Patent 79087 (ausgestellt auf G. Pforr) beschreibt ein Gerät zum Betrieb eines induktiven Plasmabrenners. Ein von einem Hochfrequenzgenerator erzeugter hochfrequenter Wechselstrom wird einer Arbeitsspule zugeführt. Die Arbeitsspule ist beweglich und mit einem Leistungskoaxialkabel am Hochfrequenzgenerator angeschlossen. Innerhalb des Isolierrohrs der Arbeitsspule wird eine induktive Plasmaflamme erzeugt.

Die europäische Patentanmeldung 0155496 (ausgestellt auf Peter Gagne) beschreibt eine Plasma-Emissionsquelle. Sie enthält einen HF-Leistungsgenerator, einen Plasmabrenner sowie Regler zur automatischen und ständigen Steuerung der vom Generator an die Lastspule geleiteten HF-Energie. Ein abstimmbarer Serien- bzw. Parallelkreis regelt die Impedanz des Leistungsgenerators bzw. der Lastspule. Jeder Kreis besitzt mindestens einen einstellbaren Kondensator und Einrichtungen zum Steuern der Kondensatoren. Diese bestehen aus primären und sekundären Motoren zum Steuern der erwähnten Kondensatoren sowie einem Detektor zur Lieferung von Eingangssignalen, die das Phasenverhältnis der HF-Spannung und des HF-Stroms widerspiegeln.

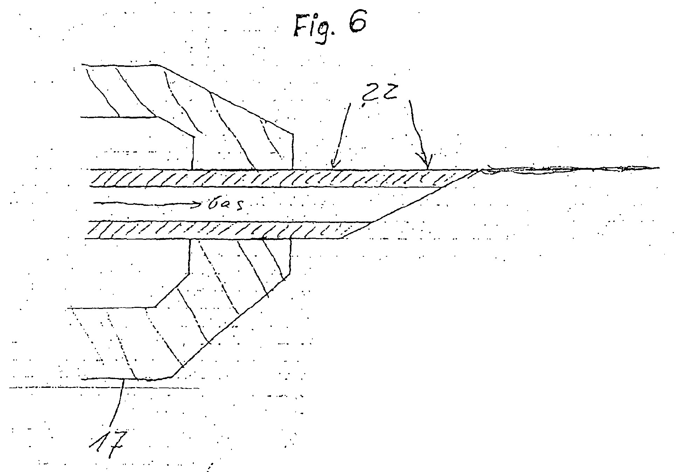

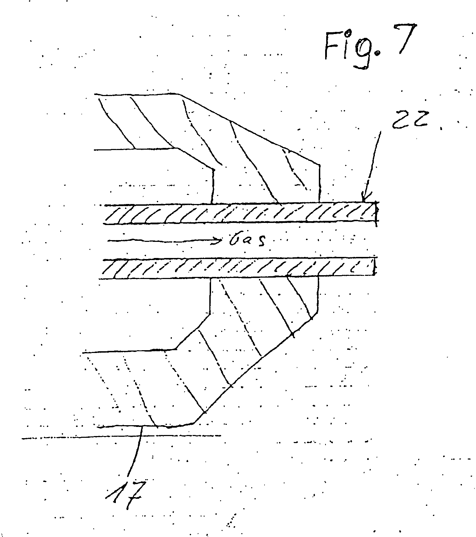

Ein Handstück für chirurgische Schnitte mit einem Plasmastrahl wird im US-Patent 4,781,175 (ausgestellt auf Birtcher) beschrieben und besonders in den Figuren 6 bis 9 dargestellt.
Konventionelle HF-Chirurgieinstrumente liefern instabile Signale mit hohen Stromspitzen von mehreren Ampere. Diese Stromspitzen konventioneller Geräte können aufgrund der hohen Potentialunterschiede zwischen der Innenseite und der Außenseite der Zellmembran auch in tiefergelegenen Gewebepartien Schäden verursachen.

Die genannten Probleme bekannter Plasmageneratoren haben grundsätzlich zu einer Einschränkung der Verwendbarkeit der elektromagnetischen Technik in der Medizin, insbesondere der Chirurgie, geführt.

Es ist Aufgabe der vorliegenden Erfindung, einen alternativen Plasmagenerator für chirurgische Anwendungen zu entwickeln, wobei dieser nicht die oben genannten Probleme konventioneller Plasmageneratoren aufweist.

Außerdem soll mit der vorliegenden Erfindung eine Verschorfung mit einem chirurgischen Kaltplasmastrahlgerät erzeugt werden, die soweit wie möglich ohne Verkohlung oder Verbrennungsprodukte aufgrund von Sauerstoffeinschlüssen ist, wie dies in Verbindung mit der Argonplasma-Methode auftreten kann.

Diese und andere Faktoren und Vorteile der vorliegenden Erfindung werden durch die folgende Beschreibung erläutert.

Die vorliegende Erfindung bezieht sich auf ein Kaltplasmagerät, das in der HF-Chirurgie zur Gerinnung von Gewebeoberflächen und zum abrasiven Entfernen von Gewebe verwendet wird.

Das in der vorliegenden Erfindung beschriebene Kaltplasmagerät enthält einen HF-Generator, einen Resonanzübertrager, ein Handstück und eine Heliumgasquelle.

Das Gerät dient zur Gerinnung von Gewebe durch Plasmaentladungen in Bereich der HF-Chirurgie. Aufbau und Funktionen sind wie folgt: ein HF-Generator sendet sein Signal an einen Resonanzübertrager. Der Resonanzübertrager transformiert das HF-Signal von ca. 350 kHz auf eine Spannung von ca. 2000 bis 3000 Volt. Der Ausgang dieses Resonanzübertragers (Sekundärwicklung) ist über eine Koaxialleitung mit integrierter Gasleitung zur Versorgung mit Helium mit einem Handstück verbunden. Der zweite Pol der Sekundärwicklung ist geerdet. Das Handstück besitzt eine Elektrodenspitze, die in einer Düse angeordnet ist, durch und um welche das Schutzgas Helium strömt. Diese Elektrodenspitze ist mit dem Leiter der Koaxialleitung verbunden, der über einen Kondensator die HF-Spannung zuführt. Wird der HF-Generator eingeschaltet, so findet eine sofortige Koronaentladung an der Elektrodenspitze innerhalb der Düse statt. Der Gasstrom treibt die Koronaentladung so durch die Düse nach außen, daß eine Flammenentladung in der Luft erfolgt. Wird diese Flamme gegen ein Gewebe gerichtet, so erfolgt eine Bogenentladung zum Gewebe, die mit einem entsprechenden Lichtbogen einhergeht. Die hohe Stromdichte am Auftreffpunkt sowie die Beschleunigung der Ladungsträger (Ionen) unmittelbar am Auftreffpunkt des elektrischen Lichtbogens bewirken eine Koagulation sowie ein Verdampfen des Gewebes, falls die Frequenz ausreichend hoch gewählt wurde.

Der HF-Strom fließt dann als dielektrischer Verschiebungsstrom von der Oberfläche des Patienten nach Masse ab, solange der Patient nicht elektrisch geerdet ist. Eine neutrale Elektrode wird nicht verwendet, und auf diese Weise wird ein Stromfluß durch den Patienten verhindert oder zumindest wesentlich verringert. Der im Handstück eingebaute Kondensator beschränkt durch seine geringe Kapazität den HF-Strom auf Werte unter 150 mA und hält somit die Leckströme unterhalb der für die HF-Chirurgie maximal zulässigen Werte. Der Resonanzübertrager wird im Gegensatz zu konventionellen HF-Chirurgiegeräten nicht durch Impulse erregt, sondern die Erregung des Resonanzübertragers findet ständig statt. Dadurch erzeugt der Resonanzübertrager ein reines Sinussignal ohne die in konventionellen HF- Chirurgiegeräten üblichen hohen Stromspitzen von mehreren Ampere, und diese Signale verursachen auch keinerlei Schädigung von tiefergelegenen Gewebepartien.

Der Schneideffekt in der HF-Chirurgie basiert auf dem Prinzip des Zellrisses. Die erzielten Schnitte sind als glatt, koagulationsreich und verschorft zu bezeichnen.

Ein glatter Schnitt in der HF-Chirurgie ist weitgehend mit einem durch ein Skalpell erzielten Schnitt vergleichbar, d.h. die Schnittflächen sind nur wenig verfärbt. Der Schnittyp des glatten Schnitts wird mit einem unmodulierten HF-Strom und bei schneller Durchführung des Schnitts erzielt.

Eine Verringerung der Schnittgeschwindigkeit hat einen Schnitt mit stärkerer Koagulation bzw. einen Schnitt mit Schorf zu Folge. Das gleiche Ergebnis wird erreicht, wenn ein pulsmodulierter HF-Strom der gleichen durchschnittlichen Leistung verwendet wird.

Die für die Durchführung eines HF-Schnitts notwendige Leistung hängt von der Form der Elektrode, dem Gewebetyp sowie dessen Widerstand ab. Ist die Leistung zu gering, findet kein Zellriss statt, und das Gewebe bleibt an der Elektrode kleben. Ist die Leistung zu hoch, können Funkenentladungen an der Elektrode und im Gewebe stattfinden, was eine Verkohlung der geschnittenen Oberflächen zur Folge hat.

Das Gewebe wird mehr oder weniger soweit erwärmt, daß für die Koagulation kein Zellriß stattfindet. Die Koagulationstemperatur liegt bei über 50 °C. Diese Temperatur führt zur Koagulation des innerhalb und außerhalb der Zelle befindlichen Zelleiweißes durch Verkochen des Gewebes. Verletzte Blutgefäße ziehen sich soweit zusammen, daß sie vollständig verschlossen werden und kein Blut austritt. Um diesen Effekt zu erzielen, muß ein elektrischer Strom das Gewebe genügend langsam erwärmen, so daß die Flüssigkeiten innerhalb und außerhalb der Zelle verdampft werden, ohne daß die Zellmembranen Schaden nehmen. Die Zellen ziehen sich aufgrund des Flüssigkeitsverlusts zusammen und die Zellwände werden zusammengeschweißt. Diese Art der Koagulation wird häufig Kontaktkoagulation genannt, weil die Elektroden in direkten Kontakt mit dem Gewebe gebracht werden. Eine Zufuhr von HF-Strom verursacht eine leichte Verfärbung des Gewebes sowie aufgrund der Eiweißgerinnung ein Ausfließen von Gewebeflüssigkeit.

Die vorliegende Erfindung besitzt einen separaten Oszillator, der für die' Frequenz des Plasmagenerators zur Erzeugung eines kalten Plasmastrahls verwendet wird; dieser Oszillator steuert eine Leistungsendstufe.

Eine Ausführung des Plasmagenerators besteht darin, daß das Ausgangssignal der als Schalter arbeitenden Endstufe der Primärwicklung des Resonanzübertragers zugeführt wird.

Eine weitere Eigenschaft des Plasmagenerators besteht darin, daß die Phasendifferenz zwischen dem Oszillatorsignal und dem Ausgangssignal der Endstufe an der Primärwicklung zur Einstellung der Oszillatorfrequenz gemessen wird.

Eine weitere Ausführung des Gegenstands der vorliegenden Erfindung enthält ein Steuersystem, mit dem die Oszillatorfrequenz durch das Steuersystem relativ zur Resonanzfrequenz des Resonanzübertragers abgestimmt wird.

Zusätzlich enthält der Plasmagenerator eine automatische Steuerung der Hochfrequenzleistung durch Veränderung der Betriebsspannung; hierbei wird die Leistung durch Multiplikation des Eingangsstroms mit der Eingangsspannung an der Endstufe ermittelt.

Ausführungsformen, die als für diese Erfindung charakteristisch angesehen werden, sind in den beigefügten Ansprüchen beschrieben. Die Erfindung selbst in ihrer Konstruktion und Wirkungsweise sowie den zusätzliche Komponenten und deren Vorzügen kann jedoch am besten durch die folgende Beschreibung der einzelnen Ausführungen in Zusammenhang mit der entsprechenden Zeichnung verstanden werden.

Die beigefügten Zeichnungen zeigen mögliche Ausführungsbeispiele der vorliegenden Erfindung:, es zeigt:
- Figur 1: zeigt ein Schaltbild einer ersten Ausführung des Plasmagenerators und des Handstücks,
- Figur 2: zeigt eine Detailansicht des Handstücks gemäß Figur 1,
- Figur 3: zeigt ein Schaltbild einer zweiten Ausführung des Plasmagenerators mit ei- nem Handstück,
- Figur 4: zeigt den Schnitt durch ein erstes Handstück,
- Figur 5: zeigt den Schnitt durch die Elektrodenspitze im Handstück,
- Figur 6: zeigt den Schnitt durch ein schräges Metallrohr mit einer Gasentladung, wobei das Metallrohr gleichzeitig als Elektrode dient,
- Figur 7: zeigt den Schnitt durch ein gerades Metallrohr mit einer Gasentladung, wobei das Metallrohr gleichzeitig als Elektrode dient,
- Figur 8: ist eine perspektivische Explosionszeichnung des Handstücks mit einer Schraubverbindung gemäß vorliegender Erfindung,
- Figur 9: ist ein seitliche Explosionszeichnung des Handstücks mit auswechselbarer Spitze,
- Figur 10: zeigt den Schnitt durch eine erste Spitze für die Endoskopie,
- Figur 11: zeigt den Schnitt durch eine erste Spitze für die Endoskopie gemäß Figur 10,
- Figur 12: zeigt den Schnitt durch eine zweite Spitze für die Endoskopie,
- Figur 13: zeigt den Schnitt durch den Resonanzübertrager,
- Figur 14: zeigt den Schnitt durch den Resonanzübertrager zusammen mit einem Ferrit- kem,
- Figur 15: ist eine perspektivische Explosionszeichnung eines Handstücks gemäß vor- liegender Erfindung, ähnlich der Konstruktion gemäß Figur 8, aber mit Steck- verbindung,
- Figur 16: ist ein Blockschaltbild des Plasmagenerators,
- Figur 17: zeigt den Anschluß und die Konstruktion des chirurgischen Plasmastrahl- Handstücks.

Der HF-Generator gemäß der vorliegenden Erfindung wird direkt mit einem Resonanzübertrager verbunden. Der HF-Generator enthält einen Schalttransistor, der die Betriebsspannung an der Primärwicklung des Resonanzübertragers periodisch unterbricht, eine automatische Einrichtung, die die Schaltfrequenz des Transistors an die Resonanzfrequenz des Resonanzübertragers anpaßt, und eine Einrichtung, mit der die elektrische Entladung am Resonanzübertrager für die automatische Steuerung und Begrenzung der Ausgangsleistung durch Verändern der Betriebsspannung gemessen werden kann.
Die Anlage gemäß vorliegender Erfindung umfaßt einen HF-Generator 3, der eine HF-Spannung mittels eines Schalttransistors 5 erzeugt, einen Resonanzübertrager 6, wobei die HF-Spannung durch eine Koaxialleitung 11 und einen Kondensator 18 mit kleiner Kapazität von ca. 5 bis 50pF an eine Elektrode 22 geleitet wird. Diese Elektrode 22 ist in einer Düse 23 am Ende des Handstücks 17 untergebracht. Gleichzeitig wird ein leicht ionisierbares Gas wie Helium von einem hier nicht beschriebenen Vorratsbehälter 2 durch eine Gaszuleitung 12 im Handstückkabel 14 des Handstücks 17 in das Handstück 17 geleitet. Der Gasanschluß 19 ist innerhalb des Handstücks mit einem Metallrohr 20 verbunden und besitzt die Gasöffnungen 21 sowie die Elektrode 22 (Figur 2). Das Gas strömt über die Gasöffnungen 21 aus der Düse 23 heraus. Wird der HF-Generator 3 eingeschaltet, so fließt der HF-Strom durch die Koaxialleitung 11, das Handstückekabel 14 und den Kondensator 18 zur Elektrode 22. Ist die HF-Spannung genügend hoch, so erfolgt zwischen der Elektrode 22 und der Gewebeoberfläche 29 des zu behandelnden Patienten eine Entladung 28. Der HF-Strom fließt als ein kapazitiver Verschiebestrom 30 vom Patienten zur Erde und von der Erdverbindung 31 zurück zum HF-Generator. Eine neutrale Elektrode, wie sie bei der herkömmlichen HF-Chirurgie benötigt wird, ist nicht erforderlich. Durch die spezifische Konstruktion des HF-Generators mit einem Resonanzübertrager 6 bildet dessen sekundäre Wicklung mit der Kapazität der Koaxialleitung 11 einen parallelen Schwingkreis und so eine nahezu sinusförmige HF-Spannung, indem der Schalttransistor 5 mit der Resonanzfrequenz des Resonanzübertragers 6 gesteuert wird. Kondensator 18 im Handstück 17 begrenzt den HF-Strom auf die für die HF-Chirurgie gültigen maximalen Werte von weniger als 150 mA (IEC 601-2-2). Das Vorhandensein des Kondensators 18, der zusammen mit der Sekundärwicklung des Resonanzübertragers 6 einen Resonanzkreis bildet, sowie die Beschränkung des HF-Stroms sind im Zusammenhang mit der vorliegenden Erfindung von großer Bedeutung.

Der verhältnismäßig geringe HF-Strom von ca. 20 bis 100 mA (je nach Kapazität des im Handstück eingebauten Kondensators 18) verursacht nur eine leichte Erwärmung in Entladungskanal. Aufgrund des sehr kleinen Auftreffpunkts des Lichtbogens auf das Gewebe (mit Auftreffflächen von wenigen Hundert Mikrometern) wird lokal eine sehr hohe Stromdichte erzeugt, die das Gewebe stark erhitzt. Diese Stromdichte sinkt bereits in sehr kleinem Abstand vom Auftreffpunkt des Lichtbogens auf Werte ab, die zu keiner nennenswerten Erhitzung des Gewebes mehr führen. Da die Hochfrequenz im wesentlichen vom Patienten als kapazitiver Verschiebestrom nach Masse hin abfließt, wird ein Stromfluß durch das Gewebe und damit eine mögliche Schädigung von tieferen Gewebeschichten vermieden.

Wird der elektrische Lichtbogen in schneller Abfolge über die Gewebeoberfläche bewegt, so erfolgt die Koagulation nur mit einer geringen Eindringtiefe. Eine punktförmige Anwendung, bei der die Entladung (der Lichtbogen) im wesentlichen auf die selbe Stelle auftrifft, führt zu einer so starken Erwärmung des Gewebes, daß das Gewebe praktisch verdampft. Mit dieser Methode kann Gewebe an der Oberfläche von Gewebe verdampft werden.

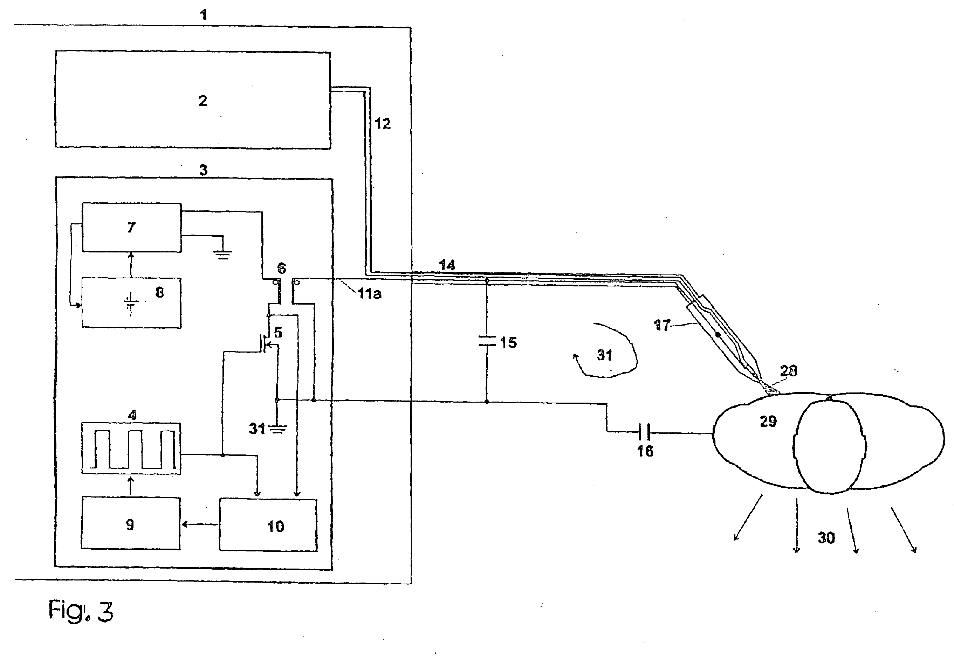

In Figur 3 ist ein Oszillator 4 abgebildet, der eine rechteckförmige Spannung an Schalttransistor 5 liefert. Dieser Schalttransistor 5 schaltet durch und legt die von der automatisch steuerbaren Spannungsquelle 8 und Leistungsaufnahme/-begrenzung 7 gelieferte Spannung periodisch an die Primärwicklung des Resonanzübertragers 6. Dieser Resonanzübertrager 6 besitzt eine Primärwicklung mit ca. 3 bis 30 Windungen sowie eine Sekundärwicklung mit 50 bis 1000 Windungen (vorzugsweise 100 bis 500), die auf einen Ferritkern mit Luftspalt aufgebracht sind. Die Kapazität 15 (Figur 3) der Koaxialleitung 11 im Handstückkabel 14 bildet zusammen mit der Sekundärwicklung des Resonanzübertragers 6 einen Resonanzkreis. Wird der Schalttransistor 5 mit der Resonanzfrequenz des Resonanzübertragers 6 angesteuert, so liefert die Sekundärwicklung des Resonanzübertragers 6 eine hohe sinusförmige HF-Spannung von 2000 bis 4000 Volt. Der HF-Strom fließt über den Kondensator 18 im Handstück 17 zur Elektrode 22. Ein leicht ionisierbares Gas wie Helium wird durch die Gaszuleitung 12 im Handstückkabel 14 zur Elektrodenspitze 22 geleitet, die in einer Düse 23 sitzt oder aus ihr hervorragen kann. Die hohe HF-Spannung erzeugt eine Plasmaentladung in Form einer elektrischen Entladung (eines Lichtbogens) 28 gegen die Gewebeoberfläche 29des Patienten. Der Patient bildet die relativ zur Masse bestehende Streukapazität 16. Der HF-Strom fließt durch diese Streukapazität und das Massepotential zurück zum Resonanzübertrager 6. Position 32 (Figur 3) zeigt den schematischen Stromverlauf.

Die Impedanz der elektrischen Entladung (des Lichtbogens) hängt von der Länge des Lichtbogens und von der Höhe des HF-Stromes ab. Die Impedanz der elektrischen Entladung (des Lichtbogens) fällt bei hohen Stromdichten im Lichtbogen stark ab. Daher muß der HF-Strom begrenzt werden. Dies geschieht durch die Impedanz des Handstückkondensators 18 (Figuren 1 und 2). Handstückkondensator 18 wird, je nach gewünschtem HF-Strom, mit einer Kapazität von vorzugsweise 10 bis 50 pF geliefert. Bei einer Generatorfrequenz von ca. 350 kHz ergibt sich damit eine Impedanz von ca. 9 bis 45 kOhm. Bei einer Spannung von 3000 Volt fließen Ströme von ca. 65 bis 330 mA. Allerdings werden nur Ströme im Bereich zwischen ca. 40 und 120 mA gemessen, da üblicherweise die Impedanz der Entladung (des Lichtbogens) 28 recht hoch ist.

Die große Resonanzgüte des Resonanzübertragers 6 erfordert eine Anregung mit der exakten Resonanzfrequenz. Die vom Handstückkondensator 18 und von der elektrischen Entladung (dem Lichtbogen) bestimmte Resonanzfrequenz beeinflußt die Resonanzfrequenz des Resonanzübertragers 6. Dies führt zu einem starken Abfall der Resonanzfrequenz zu dem Zeitpunkt, an dem die Plasmaentladung beginnt. Dieser Resonanzabfall ist je nach den Entladungsbedingungen innerhalb der Entladung (des Lichtbogens) 28 unterschiedlich. Aus diesem Grund ist eine genaue Frequenzsteuerung des Oszillators im Verhältnis zur tatsächlichen Resonanzfrequenz des Resonanzübertragers 6 notwendig. Eine Abweichung der tatsächlichen Resonanzfrequenz von der Oszillatorfrequenz ist als Änderung des Phasenwinkels zwischen dem Oszillatorsignal und dem Signal an der Primärwicklung von Resonanzübertrager 6 nachweisbar. Die Phasenvergleichsstufe 10 vergleicht die beiden Phasenwinkel im Verhältnis zueinander und liefert den tatsächlichen Wert an den automatischen Frequenzsteuerkreis 9 (Figur 3). Weicht der Phasenwinkel vom vorherbestimmten Wert ab und stimmt nicht exakt mit der Resonanzfrequenz überein, so wird die Oszillatorfrequenz durch das Steuersignal entsprechend korrigiert.

Wenn an den Lichtbogen (die Entladung) 28 eine hohe elektrische Leistung geliefert wird, so steigt auch die Leistungsaufnahme der Endstufe einschließlich Schalttransistor 5 (Figuren 1 und 3). Ein Übersteigen des Grenzwertes für den HF-Strom durch Betriebsbedingungen des Geräts, die hier nicht näher erläutert werden, führt durch ein Steuersignal zu einer Verringerung des Gleichstroms, der von der automatisch steuerbaren Spannungsquelle 8 geliefert wird.

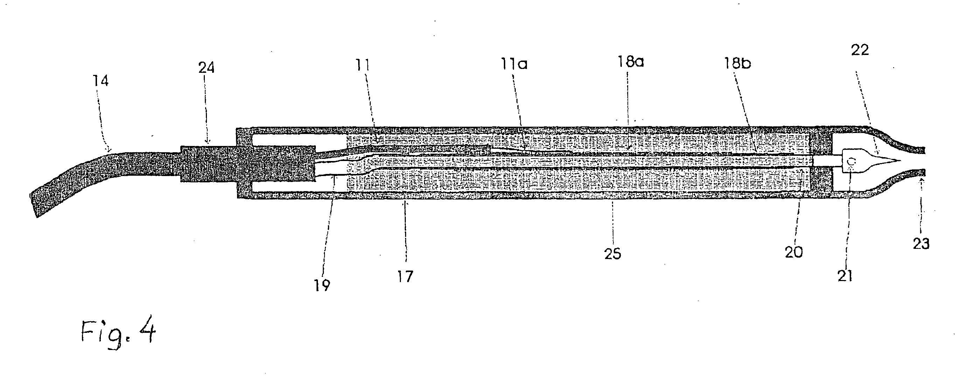

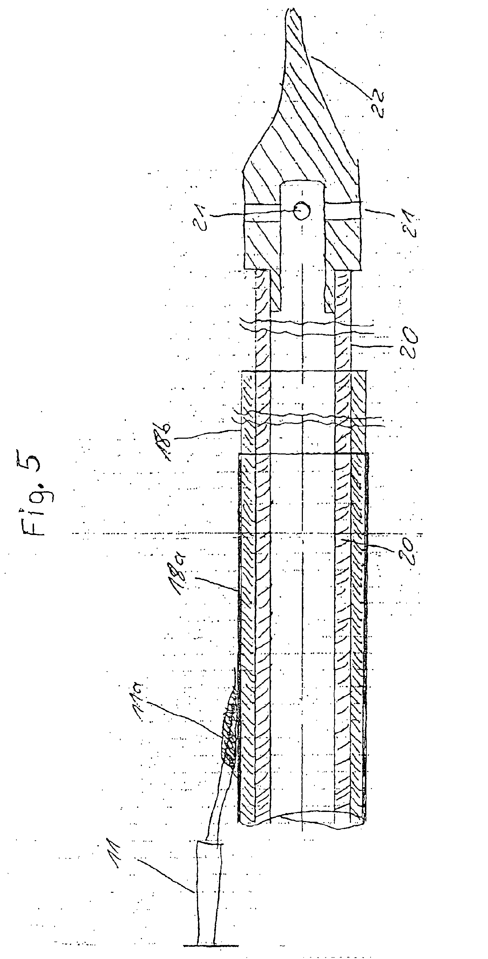

Das Handstück 17 wird in den Figuren 4 und 5 ausführlich dargestellt. Ein leicht ionisierbares Gas wie Helium strömt durch eine Zuleitung 19 vom Handstückkabel 14 in das Metallrohr 20. Das leicht ionisierbare Gas entweicht an der Gasöffnung 21 in die Düse 23 und verläßt das Handstück 17 als Strahl.

Die vom Resonanzübertrager 6 gelieferte HF-Spannung fließt durch eine Koaxialleitung 11 im Handstückkabel zu einer metallisierten Stelle 18a auf einer Isolierschicht, gebildet durch das Dielektrikum 18b. So wird z. B. ein Silikonschlauch als Dielektrikum verwendet, das auf das Metallrohr 20 geschoben wird. Eine dünne Metallfolie (z.B. eine Kupferfolie) kann als Metallisierung dienen 18a (Figur 4). Der Innenleiter des Koaxialkabels wird im Bereich 11a angeschlossen, z.B. durch Löten.

Die Metallisierung 18a auf dem Dielektrikum 18b (Figur 5) bildet zusammen mit dem Metallrohr 20 einen Kondensator (= Handstückkondensator 18), wobei dieser vorerwähnte Kondensator zur Begrenzung des Plasma-Entladungsstroms dient. Je nach Konstruktion und Größe des Metallrohrs, der Isolierschicht und der Länge der Metallisierung sind so Kapazitäten von ca. 2,5 bis 150 pF erreichbar (vorzugsweise im Bereich zwischen 5 und 50 pF). Der Strom der Plasmaentladung und somit die Plasmaleistung kann am Handstück den Erfordernissen angepaßt werden. Kapazitäten von weitaus mehr als 50 pF werden in der Regel Plasmaströme von einer Intensität erzeugen, die nicht mehr für chirurgische Anwendungen geeignet sind.

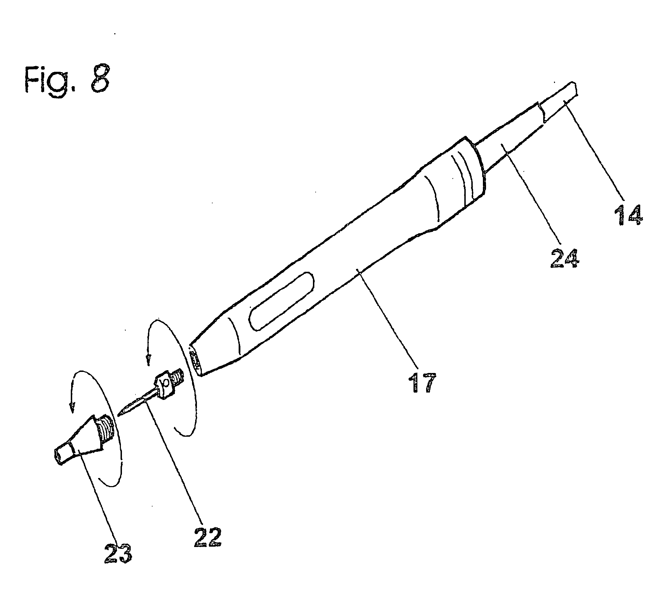

Das Handstück gemäß Figur 8 besitzt eine Konstruktion ähnlich der in den Figuren 5, 6 und 7. Die Austauschbarkeit der Düse 23 und der Elektrode 22 sind Vorteile der Ausführung gemäß Figur 8.

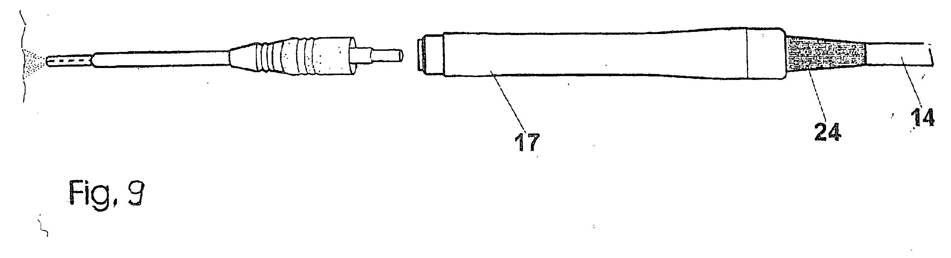

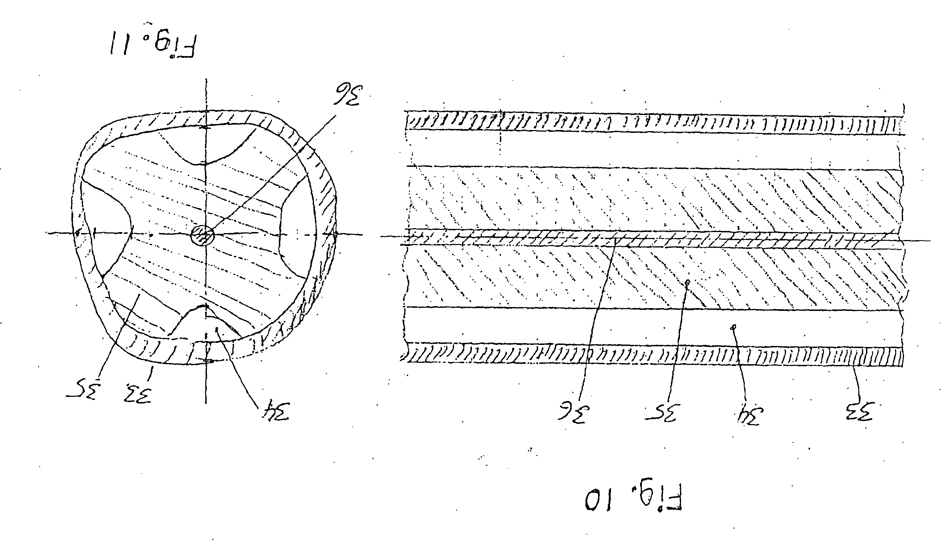

Ein Handstück mit auswechselbaren Adaptern und/oder Spitzen zeigt Figur 9. Der innere Aufbau ist grundsätzlich der gleiche wie in den Ausführungen gemäß den Figuren 5, 6, 7 und 8. Die in Figur 9 gezeigte Ausführung hat den Vorteil, daß Anwendungskomponenten verschiedener Längen und Durchmesser aufgesteckt werden können. Für die Verbindung zwischen dem Teil, das die Elektrodenspitze trägt, und dem Handstück wird eine Schraubkupplung verwendet. Die Endoskopiespitze (Figur 10) ist ein mögliches Anwendungsteil für das Handstück in Figur 9.

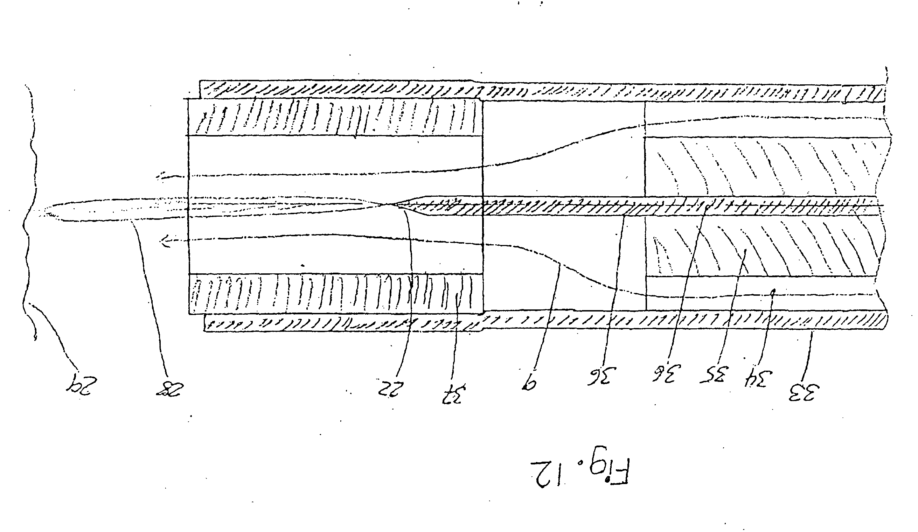

Nach den Figuren 10, 11 und 12 enthält die röhrenförmige Spitze einen Innenleiter 36 zur Weiterleitung des HF-Stroms. Dieser Innenleiter 36 sitzt in einen Kem aus isolierendem Kunststoff 35. PTFE ist besonders für diesen Zweck geeignet. Dieser Kern ist von einem Kunststoffrohr 33 umgeben. Das Gas fließt nur in Axialkanälen (siehe den Schnitt durch den Kem in Figur 10) durch das Kunststoffrohr 33. Das Gas strömt dabei durch die Axialkanäle 34, in denen eine niedrige HF-Feldstärke vorherrscht. Das Gas strömt in ein eingesetztes Keramikrohr 37. Das Ende des als Elektrodenspitze 22 ausgebildeten Innenleiters 36 liegt zentrisch in dem Keramikrohr 37. Die Entladung 28 in Richtung Gewebeoberfläche 29 geschieht von dieser Spitze aus.

Es ist aufgrund der notwendigen hochfrequenten Spannungen von Vorteil, den Hohlraum des Handstücks mit einer Isoliermasse 25 auszufüllen.

Die Kapazität zwischen dem elektrischen Leiter und der Elektrodenspitze muß für die Endoskopie so klein wie möglich sein, damit die HF-Spannung nicht zu weit absinkt, weil die geradlinige Kapazität des Leiters zur Oberfläche der Spitze oder zu den Metallteilen des Endoskops einen Spannungsteiler mit dem Handstückkondensator 18 bildet. Der Entladungsstrom kann bei Kamera-Endoskopen zu Bildstörungen führen. Die hohe Feldstärke im Bereich des Leiters ist ein weiteres Problem, da bereits hier eine lonisierung stattfinden kann. Ionisierung tritt besonders bei der Verwendung von Helium auf.

Die Konstruktion gemäß Figuren 10, 11 und 12 verringert den Effekt von Entladungsströmen auf vertretbar kleine Werte.

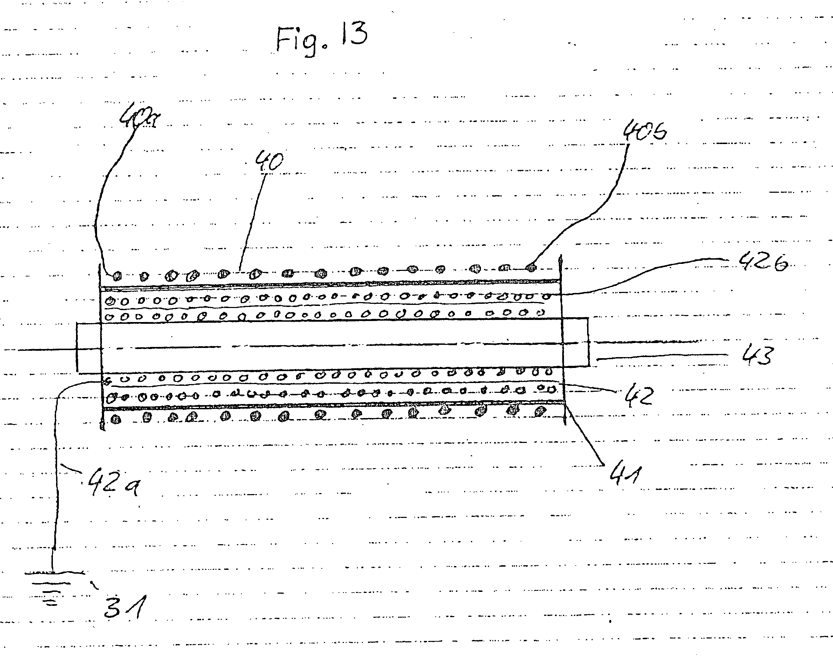

Figur 13 zeigt den Resonanzübertrager als Luftspule ohne Kern. Die Luftspule besitzt eine Primärwicklung 40 mit einer ungefähren Windungszahl von 18, hergestellt aus einem relativ dicken Kupferiackdraht von 1 bis 1,5 mm Durchmesser. Die Sekundärwicklung besteht aus ca. 450 Windungen mit einem Drahtdurchmesser von 0,2 mm. Eine Isolierfolie 41 trennt die Primär- von der Sekundärwicklung. Die Primärwicklung 40a, 40b ist gemäß Figur 1 oder Figur 3 an den Schalttransistor 5 angeschlossen. Der Anfang der Sekundärwicklung 42a liegt an Masse 31, während das Ende 42b der Sekundärwicklung mit der Koaxialleitung des Handstücks verbunden ist. Eine Resonanzfrequenz von ca. 350 kHz ergibt eine Kapazität des Handstückekabels von ca. 200 pF.

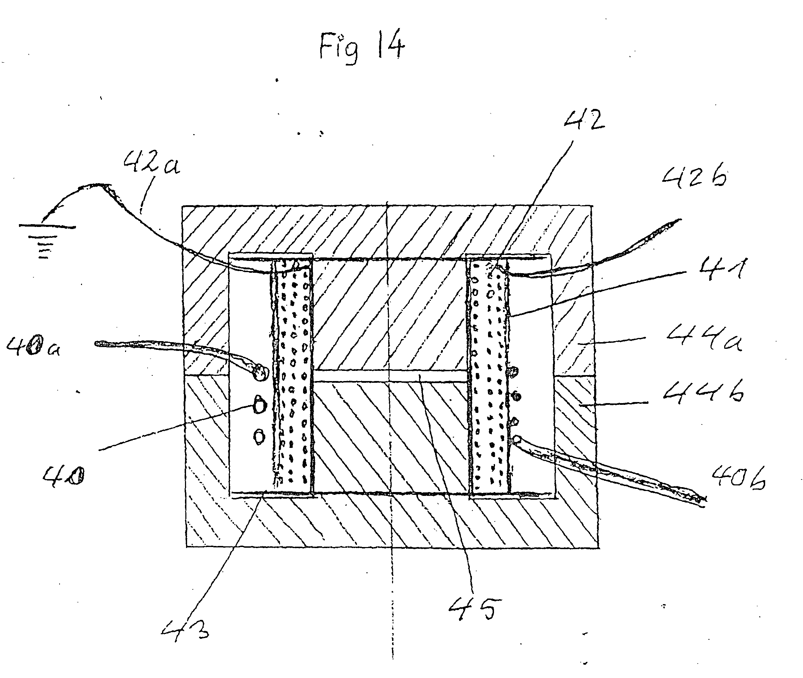

Figur 14 zeigt einen Resonanzübertrager mit einem Ferritkem. Die zwei Wicklungen 40, 42 sowie der Spulenkörper 43 sitzen hier auf einem zweiteiligen Ferritkem 44a, 44b. Der Luftspalt 45 definiert den magnetischen Widerstand und die Induktivität der Sekundärspule und damit die Resonanzfrequenz des Systems bei einer vorbestimmten Anzahl von Windungen der Sekundärspule. Der Vorteil dieser Anordnung gemäß Figur 14 im Vergleich zu der in Figur 13 besteht in der niedrigeren Anzahl der Windungen der Sekundärwicklung. Hier sind ungefähr 75 Windungen auf der Sekundärseite ausreichend. Die Primärwicklung 40 besteht aus drei Windungen Kupferlackdraht von 1 mm Durchmesser.

Die Vorteile des vorliegenden HF-Generators für chirurgische Schnitte sind der Resonanzübertrager und der im Handstück untergebrachte Kondensator. Im Gegensatz zur herkömmlichen HF-Chirurgie werden kleine Spitzenströme erzeugt. Die Konstruktion der vorliegenden Erfindung macht eine Neutralelektrode überflüssig. Der Lauf des HF-Stroms entspricht dem in den Figuren 1 und 3. Der Handstückkondensator hat vorzugsweise eine Kapazität von ca. 5 bis 50 pF zur Begrenzung des HF-Stroms. Der Oszillator wird auf eine Frequenz abgestimmt, die der Resonanzfrequenz entspricht. Der Resonanzübertrager wird vom Schalttransistor angesteuert.

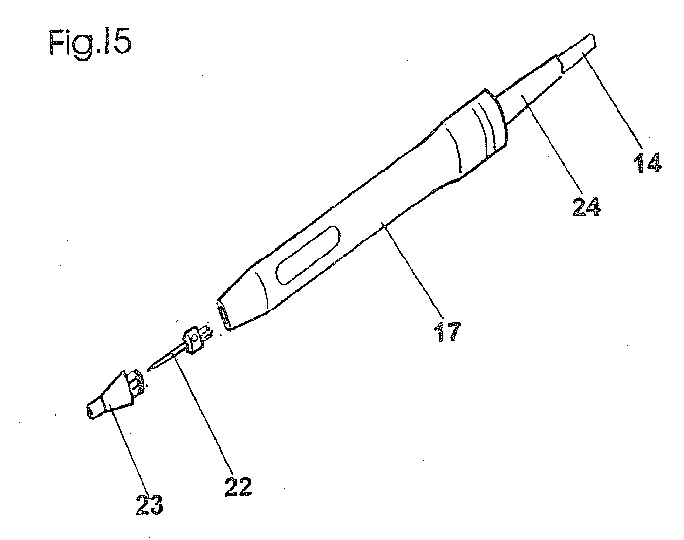

Das Handstück gemäß Figur 15 zeigt eine ähnliche Konstruktion wie in den Figuren 5, 6, 7 und 8. Die Austauschbarkeit der Düse und der Elektrode ist durch einen Schnellverschluß gegeben, der einfach und sicher zu bedienen ist. Der Schnellverschluß wird durch Führungskonturen gewährleistet, die sich in Längsrichtung des Handstücks 17 fortsetzen. Ein Verschlußmechanismus kann angebracht werden, um nach dem Einsetzen der Elektrodenspitze 22 in das Handstück 17 eine Endposition zu definieren.

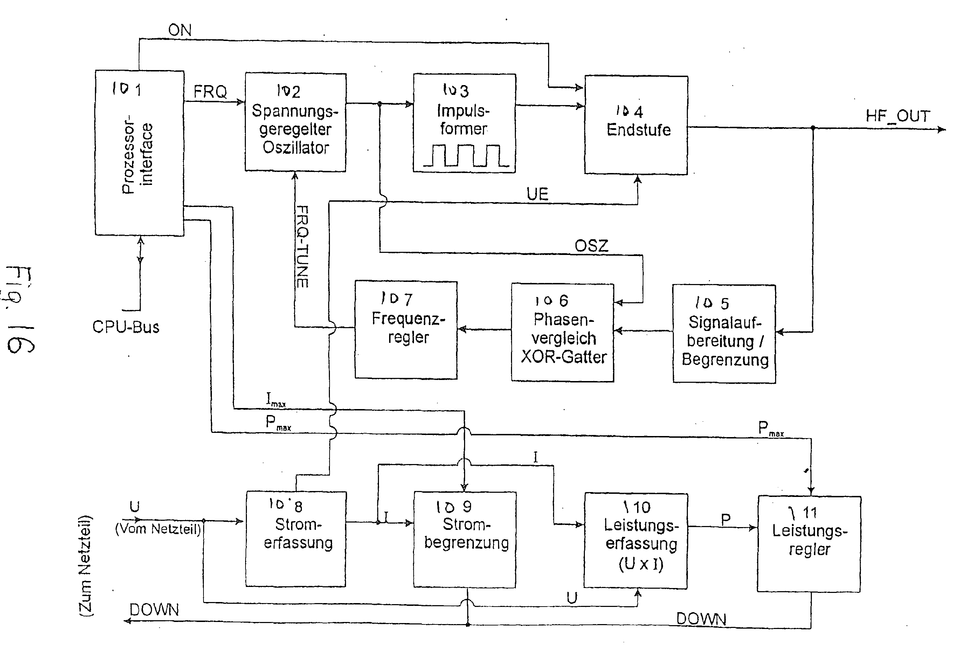

Figur 16 zeigt zusätzliche Einzelheiten eines elektronischen Schaltkreises zur Erzeugung von Hochfrequenzen für ein chirurgisches Plasmagerät. Die Frequenz wird grob durch das Signal (FRQ) über die Prozessorschnittstelle 101 gemäß Figur 16 vorgewählt Schnittstelle 101 des Prozessors, die die analogen und digitalen Signale für den Parametertransfer liefem, wird von einem Mikroprozessor (CPU-Bus) über entsprechende Software gesteuert (hier nicht näher beschrieben).

Dieses Signal (FRQ) gelangt an den spannungsgesteuerten Oszillator 102. Der Impulsumformer 103 sendet kurze Einschaltimpulse an die Endstufe 104, die wiederum periodisch die Betriebsspannung UE an die Primärwicklung des Resonanzübertragers, dargestellt durch einen Resonanzübertrager 6 (= Signal HF-OUT) legt. Das Ausgangssignal (HF-OUT) wird in Position 105 verarbeitet, d.h. das Ausgangssignal (HF-OUT) wird integriert und begrenzt und dann einem Phasenkomparator 106 zugeführt.

Phasenkomparator 106 vergleicht nun die Phasen des Ausgangssignals, das aus der Signalerzeugungsstufe 105 mit der Phase des Oszillators 102 - Signal OSZ - abgeleitet wird, und gibt dann die Phasendifferenz als eine analoge Spannung an die Frequenzsteuerung 107 weiter. Die Frequenzsteuerung vergleicht jetzt das Phasensignal mit einem vorab definierten Referenzwert und steuert die Frequenz des Oszillators 102 mit Hilfe des Signals (FRQ-TUNE) auf eine Frequenz, deren Phasenunterschied mit dem vorab bestimmten Referenzwert der Frequenz übereinstimmt. Der Resonanzübertrager wird nach diesem Vorgang auf die gewünschte Resonanzfrequenz abgestimmt.

Die Betriebsspannung U gelangt vom Netzteil durch eine Strommeßeinheit 108 an die Endstufe 104. Strommeßeinheit 108 sendet das Signal I an die Strombegrenzerstufe 109 und verhindert so eine Überlastung der Endstufe 104. Die Spannung U des Netzteils wird durch das Steuersignal DOWN entsprechend verringert, sobald der durch das Signal I-max definierte Strom überschritten wird.

Die Leistungsmeßeinheit 110 multipliziert den Ist-Strom 1a der Endstufe mit der an der Endstufe anliegenden Betriebsspannung U und liefert daraus das Ergebnis, d.h. die von Endstufe 4 aufgenommene Leistung, an die Leistungsregler 111.

Der Leistungsregler 111 vergleicht nun das Leistungssignal P mit dem von der Schnittstelle bereitgestellten vorab definierten Wert Pmax. Wird dieser überschritten, so wird die Netzteilspannung so lange durch das Steuersignal DOWN verringert, bis die vorab eingestellte Leistung erreicht ist.

Der Schalttransistor legt die Primärwicklung des Resonanzübertragers periodisch an die Versorgungsspannung. Dies muß exakt synchron zur Resonanzfrequenz des Resonanzübertragers 6 geschehen. Die Resonanzfrequenz ist richtig gewählt, wenn die Ist-Spannung an der Primärwicklung im Einschaltmoment ein entgegengesetztes Potential zur Betriebsspannung aufweist. Die Phasendifferenz zwischen dem Kontrollsignal am Schalttransistor und dem HF-Signal am Resonanzübertrager 6 kann daher als ein direktes Kriterium für die richtige Resonanzfrequenz verwendet werden.

Resonanzübertrager 6 enthält eine Primärwicklung mit wenigen Windungen sowie eine Sekundärwicklung. Die Sekundärwicklung ist mit einem Koaxialkabel des Handstückanschlusses verbunden. Die Induktivität der Sekundärwicklung und die Kapazität des Koaxialkabels bilden einen Schwingkreis, wobei der HF-Generator auf dessen Resonanzfrequenz abgestimmt werden muß. Am Ausgang des Resonanzübertragers wird eine HF-Spannung von 2000 bis 3000 Volt erreicht. Der Außenleiter des Koaxialkabels ist zusätzlich geerdet.

Die Amplitude der HF-Spannung an der Primärwicklung des Resonanzübertragers entspricht bei einer korrekten Resonanzfrequenz ungefähr der Betriebsspannung. Die automatische Steuerung der HF-Ausgangsspannung wird durch Änderung der Betriebsspannung erzielt. Eine automatische Steuerung und/oder Begrenzung der Ausgangsleistung wird durch eine direkte Messung der Entladungsleistung am Resonanzübertrager erreicht.

Zwischen HF-Generator und Resonanzübertrager befindet sich eine Überwachungsschaltung, die die erzielte Leistung der HF-Spannung und andere Parameter mißt, prüft und überwacht. Die Überwachungsschaltung schaltet das Gerät aus und gibt einen Alarm aus, wenn unzulässige Abweichungen (z.B. bei der Leistungsentladung, dem HF-Strom und anderen Parametern) erkannt werden.

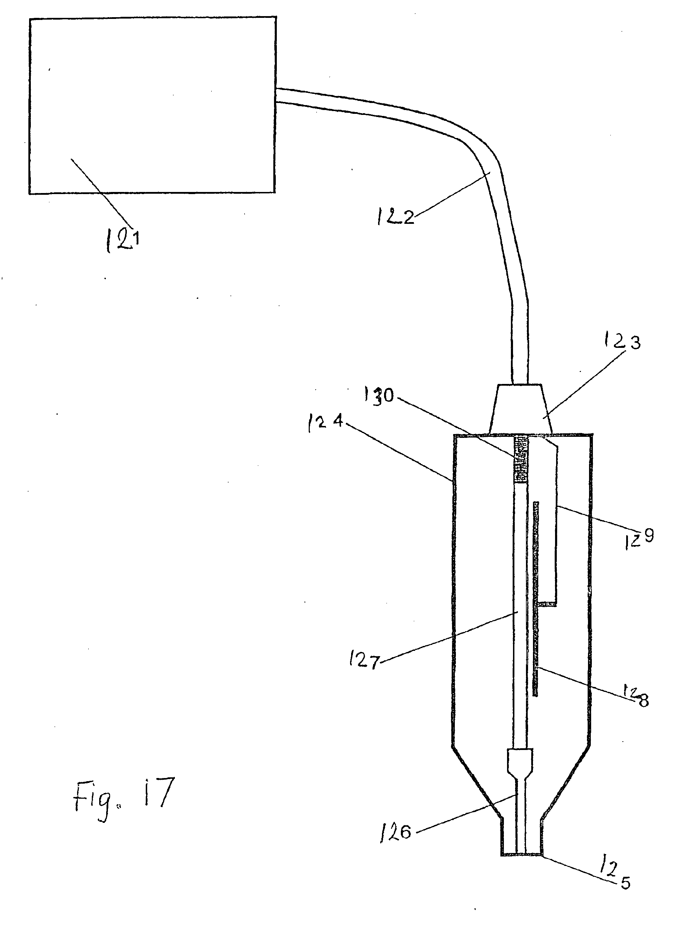

Der Anschluß des Plasma-HF-Generators an ein Handstück für chirurgische Anwendungen wird in der Ausführung aus Figur 17 gezeigt.

Ein HF-Generator 121 ist über ein Verbindungsstück 123 und ein HF-Verbindungskabel 122 mit Gasleitung an ein Handstück 124 angeschlossen.

Handstück 124 ist mit dem HF-Generator 121 über das Verbindungskabel 122 verbunden; der HF-Generator 121 umfaßt ein Koaxialkabel und eine Helium-Gasleitung sowie Steuerleitungen für die Bedientaste zum Einschalten des Geräts.

Verbindungskabel 122 ist an ein isoliertes Gasrohr 130 angeschlossen. Dieses wird zunächst durch einen ersten Kondensator (Position 127) verlängert, der eine Gasleitung besitzt. Dieser erste Kondensator 127 befindet sich zentrisch in dem länglichen, zylinderförmigen Handstück 124.

Ein Kondensator 127, 128 befindet sich im Handstück 124, zwischen der Elektrodenspitze und dem Innenleiter des Verbindungskabel 122. Dieser Kondensator 127, 128 begrenzt den maximalen HF-Strom auf Werte unterhalb einer definierten, vorab festgelegten Größe von z.B. 150 mA, welche die maximal zulässige Stromstärke für HF-Chirurgie darstellt.

Handstück 124 läuft an seinem Ende konisch zu und endet an Verbindungsstück 123; das andere Ende trägt eine Gasaustrittsöffnung/Düse 125. Der erste Kondensator 127 erstreckt sich außen an einer Elektrode 126, die in die Gasaustrittsöffnung/Düse 125 hineinragt. Ein zweiter Kondensatorteil 128 liegt dem ersten Kondensatorteil 127 gegenüber. Eine Verbindungsleitung 129 verbindet das Verbindungsstück 123 mit dem zweiten Kondensatorteil 128.

Die Elektrode 126 sowie die Gasaustrittsöffnung befinden sich innerhalb einer hitzebeständigen Düse 125. Die Gasaustrittsöffnung sowie die Elektrode können auch als Metallrohr ausgebildet werden. Die Plasmaentladung (elektrischer Lichtbogen) beginnt an dieser Elektrode. Der Kondensator kann zusammen mit der Elektrode als ein Teil konstruiert werden.

### ANWENDUNG DES HF-GENERATORS

Die Vorteile des Systems der vorliegenden Erfindung zur Koagulation von Gewebe sind eine sehr geringe Eindringtiefe der Koagulation, sehr kleine HF-Stromspitzen (und damit stark verringerter Stromfluß durch den Patienten, da dieser nicht an eine ungeerdete Elektrode angeschlossen werden muß), sowie eindeutig geringere Schädigung von Zellen in der Koagulationszone.

Natürlich kann jedes der oben beschriebenen Elemente, entweder einzeln oder in beliebiger Kombination mit anderen, auch in anderen Typen von HF-Generatoren und chirurgischen Schneidgeräten, die sich von den oben beschriebenen unterscheiden, sinnvoll eingesetzt werden.

Obgleich die Erfindung in einer Ausführung für das chirurgische HF-Schneiden gezeichnet und beschrieben worden ist, ist sie nicht auf die gezeigten Einzelheiten beschränkt, da verschiedene Modifikationen und Strukturänderungen möglich sind, ohne daß die vorliegende Erfindung im grundsätzlichen Sinn verändert werden würde.

Ohne weitere Analyse wird das oben Gesagte die Grundlagen der vorliegenden Erfindung vollständig erklären, so daß Dritte durch Anwendung allgemein zugänglichen Wissens diese ohne Schwierigkeiten für andere Anwendungsgebiete adaptieren können, ohne Funktionen auszulassen, die von jetzigen Technologiestand gesehen die wesentlichen Eigenschaften der eigentlichen oder spezifischen Aspekte der vorliegenden Erfindung darstellen.

### Positions-Nummemliste:

- 1 -: Gesamtgerät
- 2 -: Vorratsbehälter
- 3 -: HF-Generator
- 4 -: Oszillator
- 5 -: Schalttransistor
- 6 -: Resonanzübertrager
- 7 -: Leistungsaufnahme/-begrenzung
- 8 -: Spannungsquelle
- 9 -: Frequenzsteuerkreis
- 10 -: Phasenvergleichsstufe
- 11 -: Koaxialleitung
- 11 a -: Verbindungsbereich
- 12 -: Gaszuleitung
- 13 - 14 -: Handstückkabel
- 15 -: Kapazität
- 16 -: Streukapazität
- 17 -: Handstück
- 18 -: Handstückkondensator
- 18a -: Metallisierung
- 18b -: Dielektrikum
- 19 -: Zuleitung
- 20 -: Metallrohr
- 21 -: Gasöffnungen
- 22 -: Elektrodenspitze
- 23 -: Düse
- 24 - 25 -: Isoliermasse
- 26 - 27 - 28 -: Entladung (Lichtbogen)
- 29 -: Gewebeoberfläche
- 30 -: Verschiebestrom
- 31 -: Erdverbindung
- 32 -: schematischer Stromveriauf
- 33 -: Kunststoffrohr
- 34 -: Axialkanal
- 35 -: Kunststoff

- 36 -: Innenleiter
- 37 -: Keramikrohr
- 38 - 39 - 40 -: Primärwicklung
- 40a -: Primärwicklung (Anfang)
- 40b -: Primärwicklung(Ende)
- 41 -: Isolierfolie
- 42 -: Sekundärwicklung
- 42a -: Sekundärwicklung (Masseseite)
- 42b -: Sekundärwicklung (Koaxialseite)
- 43 -: Spulenkörper
- 44a -: Ferritkernhälfte
- 44b -: Ferritkernhälfte
- 45 -: Luftspalt

- 101 -: Prozessorschnittstelle
- 102 -: Oszillator
- 103 -: Impulsumformer
- 104 -: Endstufe
- 105 -: Signalerzeugungsstufe
- 106 -: Phasenkompensator
- 107 -: Frequenzsteuerung
- 108 -: Strommesseinheit
- 109 -: Strombegrenzer
- 110 -: Leistungsmesseinheit
- 111 -: Leistungsregler

- 121 -: HF-Generator, Gerät
- 122 -: Verbindungskabel
- 123 -: Verbindungsstück
- 124 -: Handstück
- 125 -: Gasaustrittsöffnung
- 126 -: Elektrode
- 127 -: 1. Kondensatorteil
- 128 -: 2. Kondensatorteil
- 129 -: Verbindungsleitung
- 130 -: Gasrohr

## Patentansprüche

1. Ein Plasmagenerator zur Erzeugung eines kalten Plasmastrahls, bestehend aus:
einem frequenzvariablen Oszillator 4;
einer Leistungsendstufe, die an den frequenzvariablen Oszillator 4 angeschlossen ist und durch den sie gesteuert wird.

2. Der Plasmagenerator gemäß Anspruch 1, mit einer als Schalter betriebenen Leistungsendstufe, sowie mit
einem Resonanzübertrager 6 mit Primärwicklung 40 und Sekundärwicklung 42, an dessen Primärwicklung 40 das Ausgangssignal der Leistungsendstufe gelegt wird.

3. Der Plasmagenerator gemäß Anspruch 2, mit folgenden weiteren Elementen:
Meßeinrichtungen mit Ausgang, angeschlossen an die Primärwicklung 40 des Resonanzübertragers 6, zur Messung der Phasendifferenz zwischen dem Oszillatorsignal und einem Ausgangssignal der Leistungsendstufe durch die Meβeinrichtungen an der Primärwicklung 40; der Ausgang der Meßeinrichtungen ist an einen Eingang des frequenzvariablen Oszillators 4 angeschlossen und steuert dessen Ausgangsfrequenz.

4. Der Plasmagenerator gemäß Anspruch 2, der zusätzlich folgendes enthält:
eine an den frequenzvariablen Oszillator 4 angeschlossene Steuereinheit, die dessen Frequenz relativ zur Resonanzfrequenz des Resonanzübertragers 6 abstimmt.

5. Der Plasmagenerator gemäß Anspruch 1, der zusätzlich folgendes enthält:
Vorrichtungen zur Veränderung der Betriebsspannung zur Steuerung der abgegebenen HF-Leistung durch Änderung der Betriebsspannung, und
eine an die Leistungsendstufe angeschlossene Leistungsmeßeinrichtung, die die Leistung der Leistungsendstufe durch Multiplikation eines Eingangsstroms mit einer Eingangsspannung am Eingang der Leistungsendstufe mißt.

6. Der Plasmagenerator gemäß Anspruch 5, der zusätzlich folgendes enthält:
Vorrichtungen zur Messung der von der Leistungsendstufe gelieferten HF-Leistung, angeschlossen an die Leistungsendstufe.

7. Der Plasmagenerator gemäß Anspruch 2, der zusätzlich folgendes enthält:
Vorrichtungen zur Messung der vom Resonanzübertrager 6 gelieferten HF-Leistung,
angeschlossen an den Resonanzübertrager.

8. Der Plasmagenerator gemäß Anspruch 1, der zusätzlich folgendes enthält:
ein Handstück 17 mit einem ersten Ende;
ein HF-Anschlußkabel mit Gasschlauch,
angeschlossen an die Leistungsendstufe und an das erste Ende des Handstücks 17.

9. Der Plasmagenerator gemäß Anspruch 8, dessen Handstück folgendes enthält:
ein isoliertes Gasrohr, angeschlossen an das HF-Anschlußkabel;
ein erstes Kondensatorteil mit einem Gasrohr;
das isolierte Gasrohr ragt in das erste Kondensatorteil (mit Gasrohr) hinein;
das erste Kondensatorteil sitzt zentrisch in dem länglichen, zylinderförmigen Handstück;
eine Elektrode mit Spitze, am zweiten Ende des Handstücks gegenüber dem ersten Ende;
ein Kondensator in dem Handstück, der an die Spitze der Elektrode und an das HF-Anschlußkabel angeschlossen ist.

10. Der Plasmagenerator gemäß Anspruch 9, dessen Handstück folgendes enthält:
eine Gasöffnung 21 am zweiten Ende des Handstücks,
das an seinem zweiten Ende konisch zuläuft;
eine Elektrodenspitze 22 innerhalb der Gasöffnung; das erste Kondensatorteil erstreckt sich außen an der Elektrodenspitze 22;
ein zweites Kondensatorteil gegenüber dem ersten Kondensatorteil;
eine Kondensatoranschlußkabel, angeschlossen an das Anschlußkabel und
das zweite Kondensatorteil.

11. Der Plasmagenerator gemäß Anspruch 10, in dem das erste Kondensatorteil und das zweite Kondensatorteil einen maximalen HF-Strom auf Werte unterhalb eines Sollwerts von ca. 40 bis 2000 mA beschränken.

12. Der Plasmagenerator gemäß Anspruch 8, dessen Handstück die Form eines länglichen Zylinders besitzt.
